(19) Europäisches Patentamt
European Patent Office
Office européen des brevets

(11) Veröffentlichungsnummer: **0 046 184**
**B1**

(12) # EUROPÄISCHE PATENTSCHRIFT

(45) Veröffentlichungstag der Patentschrift: **23.11.83**

(51) Int. Cl.³: **C 07 C 119/048,** C 07 C 118/04 // C07C127/15, A01N47/28

(21) Anmeldenummer: **81105376.8**

(22) Anmeldetag: **10.07.81**

(54) **Neue Aryloxyalkylisocyanate und Verfahren zu ihrer Herstellung.**

(30) Priorität: **16.08.80 DE 3030996**

(43) Veröffentlichungstag der Anmeldung: **24.02.82 Patentblatt 82/8**

(45) Bekanntmachung des Hinweises auf die Patenterteilung: **23.11.83 Patentblatt 83/47**

(84) Benannte Vertragsstaaten: **BE CH DE FR GB LI**

(56) Entgegenhaltungen:
**LU - A - 65 879**

**CHEMICAL ABSTRACTS, Band 90, Nr. 21, 21. Mai 1979, Seite 554, Nr. 168027w Columbus, Ohio, U.S.A. A.D. SINITSA et al.: "Thermal splitting of N-trimethylsilyl-N-(2,2,2-trichloro-1-alkoxy(aroxy)) ethylureas and -urethanes"**
**TETRAHEDRON LETTERS, Nr. 33, 1977, Seiten 2835-2838, Pergamon Press G.B. R. BONJOUKLIAN et al.: "Ansa macrolide syntheis II. A convergent approach to maytansine"**
**CHEMICAL ABSTRACTS, Band 94, Nr. 23, 8. Juni 1981, Seite 615, Nr. 191864h Columbus, Ohio, U.S.A. YA. G. BAL'ON: "Reactivity of N,N-dichlorourethanes. XII. Reaction of N,N-dichlorourethane with alpha-bromostyrene"**

(73) Patentinhaber: **BASF Aktiengesellschaft, Carl-Bosch-Strasse 38, D-6700 Ludwigshafen (DE)**

(72) Erfinder: **Husslein, Gerd, Dr., Herrenbergstrasse 2, D-6702 Bad Duerkheim (DE)**
Erfinder: **Rohr, Wolfgang, Dr., In der Dreispitz 13, D-6706 Wachenheim (DE)**

(56) Entgegenhaltungen: (Fortsetzung)
**CHEMICAL ABSTRACTS, Band 81, Nr. 17, 28. Oktober 1974, Seiten 477, Nr. 104928x Columbus, Ohio, U.S.A. L.I. SAMARAI et al.: "Anionotropic reaction of alpha-aryloxy (arylthio) alkyl isocyanates"**
**SYNTHESIS, Nr. 10, Oktober 1972, Seiten 551-553 H.R. KRICHELDORF: "Reaktionen mit Silylazides; 1. Mitteilung. Synthese von Isocyanaten aus Carbonsäurechloriden und Trimethylsilylazid"**

EP 0 046 184 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neue Aryloxyalkylisocyanate und Verfahren zu ihrer Herstellung

Die Erfindung betrifft neue Aryloxyalkylisocyanate und ein Verfahren zur Herstellung von Aryloxyalkylisocyanaten durch Umsetzung von 2-Aryloxyessigsäureverbindungen mit einem Trialkylsilylazid.

Es ist bekannt, dass sich Alkoxyalkylisocyanate durch Addition von Isocyansäure an Vinyläther (J. prakt. Chem. 320 [1978], Seite 491-492), durch Umsetzung von α-Halogenalkyläthern mit Alkali- oder Erdalkali-cyanaten in stark polaren Lösungsmitteln (DE-OS 12 05 087) oder durch Thermolyse von Alkoxyalkyldioxazolonen (J. Org. Chem. 39 [1974], Seiten 2472–2473) herstellen lassen. Alkancarbonsäurechloride lassen sich mit Trimethylsilylazid zu Alkylisocyanaten überführen. Die Darstellung von Phenoxymethylisocyanat gelingt dagegen nicht, da eine Kondensation zur Trisphenoxymethylcyanursäure erfolgt (Synthesis 1972, Seiten 551 bis 553).

Es wurde nun gefunden, dass man Aryloxyalkylisocyanate der Formel

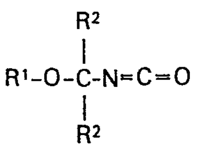

(I),

worin $R^1$ einen aromatischen Rest bezeichnet, die einzelnen Reste $R^2$ gleich oder verschieden sein können und jeweils für ein Wasserstoffatom oder einen Alkylrest mit 1 bis 18 Kohlenstoffatomen, einen cycloaliphatischen Rest, einen Aralkylrest mit 7 bis 12 Kohlenstoffatomen, oder aromatischen Rest stehen, wobei, wenn $R^1$ einen Phenylrest und ein Rest $R^2$ ein Waserstoffatom oder eine Phenylgruppe bedeuten oder wenn $R^1$ einen p-Tolylrest, p-Chlorphenylrest, p-Nitrophenylrest, 2,4,6-Trijodphenylrest, 2-Naphthylrest und ein Rest $R^2$ einen Phenylrest bezeichnen, die beiden Reste $R^2$ dann verschieden sind, vorteilhaft erhält, wenn man 2-Aryloxyessigsäureverbindungen der Formel

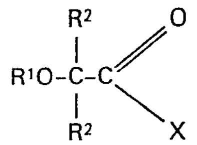

(II),

worin $R^1$ und $R^2$ die vorgenannten Bedeutungen besitzen und X ein Halogenatom, den Rest

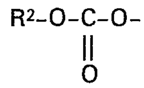 oder den Rest 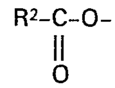

bezeichnet, mit einem Trialkylsilylazid bei einer Temperatur von −25 bis +180°C umsetzt und aus dem Reaktionsgemisch den Endstoff I in üblicher Weise, z.B. durch fraktionierte Destillation, abtrennt.

Weiterhin wurden die neuen Aryloxyalkylisocyanate der Formel

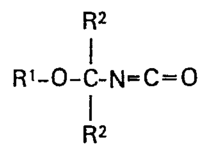

(I),

worin $R^1$ einen aromatischen Rest bezeichnet, die einzelnen Reste $R^2$ gleich oder verschieden sein können und jeweils für ein Wasserstoffatom oder einen Alkylrest mit 1 bis 18 Kohlenstoffatomen, einen cycloaliphatischen Rest, einen Aralkylrest mit 7 bis 12 Kohlenstoffatomen oder aromatischen Rest stehen, wobei, wenn $R^1$ einen Phenylrest und ein Rest $R^2$ ein Wasserstoffatom oder eine Phenylgruppe bedeuten oder, wenn $R^1$ einen p-Tolylrest, p-Chlorphenylrest, p-Nitrophenylrest, 2,4,6-Trijodphenylrest, 2-Naphthylrest und ein Rest $R^2$ einen Phenylrest bezeichnen, die beiden Reste $R^2$ dann verschieden sind, gefunden.

Verwendet man 2-(2′,4′-Dichlorphenoxy)-essigsäurechlorid und Trimethylsilylazid als Ausgangsstoffe, kann der Reaktionsablauf des erfindungsgemässen Verfahrens durch folgendes Formelschema wiedergegeben werden:

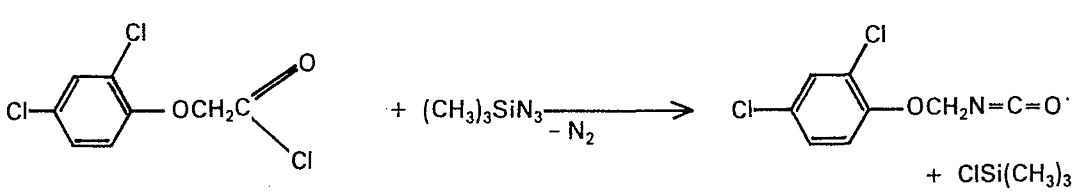


Im Hinblick auf die bekannten Verfahren liefert das Verfahren nach der Erfindung auf einfachem und wirtschaftlichem Wege die neuen 2-Aryloxyalkylisocyanate in guter Ausbeute und Reinheit. Alle diese vorteilhaften Ergebnisse sind im Hinblick auf den Stand der Technik überraschend.

Bevorzugte Ausgangsstoffe II und dementsprechend bevorzugte Endstoffe I sind solche, in deren Formeln $R^1$ einen durch 1, 2 oder 3 Chloratome, Bromatome, Alkylgruppen und/oder Alkoxygruppen mit jeweils 1 bis 6 Kohlenstoffatomen substituierten Phenylrest oder einen unsubstituierten oder in vorgenannter Weise substituierten Naphthylrest oder Phenoxyphenylrest bezeichnet, die einzelnen Reste $R^2$ gleich oder verschieden sein können und jeweils für ein

Wasserstoffatom, einen Alkylrest mit 1 bis 18, insbesondere 1 bis 8 Kohlenstoffatomen, einen Cycloalkylrest mit 5 bis 8 Kohlenstoffatomen, einen Aralkylrest oder Alkylarylrest mit 7 bis 12 Kohlenstoffatomen, einen Phenylrest stehen und X ein Chloratom oder ein Bromatom bedeutet. Die vorgenannten Reste können noch durch unter den Reaktionsbedingungen inerte Atome oder Gruppen, z.B. Alkylgruppen oder Alkoxygruppen mit jeweils 1 bis 4 Kohlenstoffatomen, Nitrogruppen, Cyangruppen, Arylreste substituierende Jodatome, Fluoratome, Chloratome, Bromatome und/oder Trifluormethylgruppen, substituiert sein.

So kommen folgende Ausgangsstoffe II in Betracht: In 2-Stellung einfach oder gleich oder verschieden zweifach durch die Methyl-, Äthyl-, n-Propyl-, iso-Butyl-, sek.-Butyl-, tert.-Butyl-, n-Pentyl-, 2′-Pentyl-, 3′-Pentyl-, 4′-Methyl-3′-pentyl-, 1′-Chloräthyl-, Trifluormethyl-, Trichlormethyl-, Difluormethyl-, Nitromethyl-, Cyanomethyl-, 2′-Chloräthyl-, 1′-Chlorpropyl-, 2′-Chlorpropyl-, 1′-Chlor-2′-propyl-, 1′-Fluoräthyl-, 2′-Fluoräthyl-, 1′-Fluor-2-propyl-, 3′-Brombutyl-, 1′,1′,1′-Trifluorisopropyl-, Methoxyäthyl-, Methoxyisopropyl-, Äthoxy-tert.-butyl-, Methoxy-tert.-butyl-, Cyclopentyl-, Cyclohexyl-, 2′-Chlorcyclohexyl-, 2′-(Trifluormethyl)-cyclohexyl-, Benzyl-, Phenyl-, α-Phenyläthyl-gruppe, durch eine in o-, m-, p-Stellung einfach oder in 2″,4″-, 2″,3″-, 2″,6″-, 2″,5″-, 4″,5″-Stellung gleich oder unterschiedlich 2fach durch Chloratome, Bromatome, Fluoratome, Methyl-, Äthyl-, Propyl-, iso-Propyl-, Butyl-, iso-Butyl-, sek.-Butyl-, tert.-Butyl-, Methoxy-, Äthoxy-, Propoxy-, iso-Propoxy-, Butoxy-, iso-Butoxy, sek.-Butoxy-, tert.-Butoxy-, Phenyl-, Trifluormethyl-gruppen substituierte Phenylgruppe substituierte 2-Phenoxyessigsäurechloride; vorgenannten Säurechloriden homologe Säureanhydride mit 2 gleichen Acylresten; gemischte Säureanhydride mit vorgenannten Acylgruppen und davon unterschiedlichen Acylgruppen, zweckmässig mit den Methyl-, Äthyl-, Propyl-, iso-Propyl-, Butyl-, iso-Butyl-, sek.-Butyl-, tert.-Butyl-, Cyclohexyl-, Cyclopentyl-, Phenyl-, Benzyl-carbonylgruppen; vorgenannten Säurechloriden entsprechende Anhydride mit Kohlensäuremonoestern; den vorgenannten Säurechloriden homologe unsubstituierte oder in vorgenannter Weise substituierte Säurebromide oder -jodide; homologe in vorgenannter Weise substituierte in 2′-, 3′-, 4′-, 5′-, 6′-Stellung am Phenylkern einfach oder gleich oder verschieden 2fach oder 3fach durch Fluoratome, Chloratome, Bromatome, Trifluormethyle-, Methyl-, Äthyl-, Propyl-, iso-Propyl-, Butyl-, iso-Butyl-, sek.-Butyl-, tert.-Butyl-, Methoxy-, Äthoxy-, Propoxy-, iso-Propoxy-, Butoxy-, iso-Butoxy-, sek.-Butoxy-, tert.-Butoxy-, Nitro-, Phenylgruppen substituierte 2-Phenoxyverbindungen.

Die Ausgangsstoffe II werden zweckmässig aus Aryloxyalkancarbonsäuren der Formel III

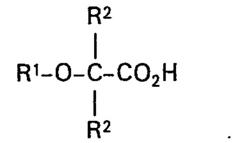

(III)

oder deren Salzen mit Halogenierungsmitteln, beispielsweise Thionylchlorid, Phosgen, Phosphorpentachlorid oder Acylierungsmitteln, beispielsweise Acetylchlorid, Acetanhydrid, Diketen, Benzoylchlorid, Chlorameisensäuremethylester, in üblicher Weise hergestellt (Houben-Weyl, Methoden der Organischen Chemie, Band VII, Seiten 463 bis 483).

Besonders bevorzugte Ausgangsstoffe II sind: 2-(2′,4′-Dichlorphenoxy)-propionsäurechlorid, 2-(4′-Chlorphenoxy)-propionsäurechlorid, 2-(3′-Trifluormethylphenoxy)-propionsäurechlorid, 2-(2′,4′-Dichlorphenoxy)-buttersäurechlorid, 2-(4′-Chlorphenoxy)-buttersäurechlorid, 2-(4′-Fluorphenoxy)-propionsäurechlorid, 2-(2′-Brom-4′-chlorphenoxy)-propionsäurechlorid, 2-(2′,4′-Dichlorphenoxy)-essigsäurechlorid, 2-(2′,4′,5′-Trichlorphenoxy)-propionsäurechlorid, 2-(4′-Phenylphenoxy)-propionsäurechlorid, 2-(4′-Nitrophenoxy)-propionsäurechlorid, 2-(4′-Nitrophenoxy)-propionsäurechlorid, 2-(2′,4′-Dichlorphenoxy)-iso-valeriansäurechlorid, 2-(3′-Trifluormethylphenoxy)-essigsäurechlorid, 2-(2′,4′-Dibromphenoxy)-essigsäurechlorid, 2-(4′-Chlorphenoxy)-essigsäurechlorid, 2-(3′,5′-Dimethylphenoxy)-essigsäurechlorid, 2-(4′-Fluorphenoxy)-essigsäurechlorid, 2-(2′-Brom-4′-chlorphenoxy)-essigsäurechlorid, 2-(2′-4′-Dichlorphenoxy)-essigsäurebromid, 2-(2′,4′,5′-Trichlorphenoxy)-essigsäurechlorid, 2-(4′-Nitrophenoxy)-essigsäurechlorid, 2-(3′-Trifluormethylphenoxy)-essigsäurebromid.

Als Trialkylsilylazide werden in der Regel solche der Formel

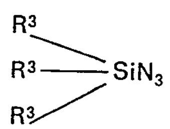

(IV)

worin die einzelnen Reste $R^3$ gleich oder verschieden sein können und jeweils für einen aliphatischen Rest, vorteilhaft für einen Alkylrest mit 1 bis 18, insbesondere 1 bis 8 Kohlenstoffatomen, stehen. Die Ausgangsstoffe IV können auch in situ gebildet werden, z.B. durch die Umsetzung von Alkaliaziden wie beispielsweise Natriumazid mit Halogentrialkylsilanen wie beispielsweise Chlortrimethylsilan, gegebenenfalls in einem ersten Schritt oder in Gegenwart einer Verbindung der Formel II. Die Umsetzung mit einer Verbindung der Formel II wird mit einer stöchiometrischen Menge oder einem Überschuss an Trialkylsilylazid, vorteilhaft in einer Menge von 1 bis 3, vorzugsweise von 1,1 bis 1,5 Mol Trialkylazid, bezogen auf den Ausgangsstoff II, durchgeführt.

Die Umsetzung wird bei einer Temperatur von −25 bis +180°C, vorzugsweise von 20 bis 150°C,

insbesondere von 30 bis 130°C, drucklos oder unter Druck, kontinuierlich oder diskontinuierlich durchgeführt. Zweckmässig verwendet man unter den Reaktionsbedingungen inerte Lösungsmittel. Als Lösungsmittel kommen z.B. in Frage: aromatische Kohlenwasserstoffe, z.B. Toluol, Äthylbenzol, o-, m-, p-Xylol, Isopropylbenzol, Methylnaphthalin; Halogenkohlenwasserstoffe, insbesondere Chlorkohlenwasserstoffe, z.B. Tetrachloräthylen, 1,1,2,2- oder 1,1,1,2-Tetrachloräthan, Amylchlorid, Dichlorpropan, Methylenchlorid, Dichlorbutan, Isopropylbromid, Chloroform, Tetrachlorkohlenstoff, 1,1,1- oder 1,1,2-Trichloräthan, Trichloräthylen, Pentachloräthan, 1,2-Dichloräthan, 1,1-Dichloräthan, n-Propylchlorid, 1,2-cis-Dichloräthylen, n-Butylchlorid, 2-, 3- und iso-Butylchlorid, Chlorbenzol, o-, p- und m-Dichlorbenzol, o-, m-, p-Chlortoluol, 1,2,4-Trichlorbenzol; Äther, z.B. Äthylpropyläther, Methyltert.-butyläther, n-Butyläthyläther, Di-n-butyläther, Diisobutyläther, Diisoamyläther, Diisopropyläther, Cyclohexylmethyläther, Diäthyläther, Äthylenglykoldimethyläther, Tetrahydrofuran, Dioxan; Ester wie Methylacetat, n-Propylacetat, Methylpropionat, Butylacetat, Äthylformiat, Phthalsäuremethylester, Benzoesäuremethylester, Essigester, Phenylacetat; Nitrile wie Acetonitril, Butyronitril, Isobutyronitril, Benzonitril, m-Chlorbenzonitril; Sulfoxide wie Dimethylsulfoxid, Diäthylsulfoxid, Dimethylsulfon, Diäthylsulfon, Methyläthylsulfon, Tetramethylensulfon; aliphatische oder cycloaliphatische Kohlenwasserstoffe, z.B. Heptan, α-Pinen, Pinan, Nonan, Benzinfraktionen innerhalb eines Siedepunktintervalls von 70 bis 190°C, Cyclohexan, Methylcyclohexan, Dekalin, Petroläther, Hexan, Ligroin, 2,2,4-Trimethylpentan, 2,2,3-Trimethylpentan, 2,3,3-Trimethylpentan, Octan; N,N-disubstituierte Säureamide wie Dimethylformamid, Dimethylacetamid; tertiäre organische Amine wie Pyridin, Picolin, Lutidin, Tributylamin; und entsprechende Gemische. Zweckmässig verwendet man das Lösungsmittel in einer Menge von 100 bis 10 000 Gewichtsprozent, vorzugsweise von 500 bis 2000 Gewichtsprozent, bezogen auf Ausgangsstoff II.

Die Reaktion kann wie folgt durchgeführt werden: Ein Gemisch von Ausgangsstoff II und IV, gegebenenfalls zusammen mit Lösungsmittel, wird während 0,5 bis 20 Stunden bei der Reaktionstemperatur gehalten. Im allgemeinen vermischt man den Ausgangsstoff II und IV bei −20 bis +30°C mit einem Verdünnungsmittel und lässt bei diesen Temperaturen während 10 Minuten bis 15 Stunden, vorzugsweise 10 Minuten und 12 Stunden, vorreagieren und beendigt die Reaktion dann bei Temperaturen von 50 bis 180°C zu den Endstoffen I. Eine besonders bevorzugte Verfahrensvariante besteht darin, dass man den Ausgangsstoff II und Lösungsmittel mit Trimethylsilylazid vermischt und durch die Anwendung von Temperaturen von +40 bis +150°C das gebildete Säureazid in situ thermolysiert. Aus dem Reaktionsgemisch wird der Endstoff I in üblicher Weise, z.B. durch fraktionierte Destillation, abgetrennt.

Die nach dem Verfahren der Erfindung herstellbaren Endstoffe I sind wertvolle Ausgangsstoffe für die Herstellung von Pharmazeutika, Pflanzenschutzmitteln und Farbstoffen. Bezüglich der Verwendung wird auf die vorgenannten Veröffentlichungen verwiesen.

Die in den folgenden Beispielen aufgeführten Teile bedeuten Gewichtsteile.

Beispiel 1

Zu einer Lösung von 144 Teilen 2′,4′-Dichlorphenoxyessigsäurechlorid in 300 Teilen trockenem Dioxan gibt man auf einmal 84 Teile Trimethylsilylazid. Man erwärmt nun das Gemisch während 10 Minuten langsam bis 60°C und hält das Reaktionsgemisch während 4 Stunden auf 75°C und dann erwärmt man noch 45 Minuten auf 105°C, lässt nach Beendigung der Stickstoffentwicklung abkühlen, zieht das Lösungsmittel im Vakuum ab und destilliert den Rückstand. Man erhält 110 Teile (84% der Theorie) 2′,4′-Dichlorphenoxymethylsisocyanat vom Kp 97 bis 99°C/0,4 mbar, $n_D^{23} = 1{,}5501$.

Beispiel 2

Zu einer Lösung von 25,4 Teilen 2-(2′,4′-Dichlorphenoxy)-propionsäurechlorid in 150 Teilen Xylol gibt man 21 Teile Trimethylsilylazid und erwärmt das Gemisch während 60 Minuten auf 105°C. Man hält das Gemisch noch eine Stunde bei dieser Temperatur, zieht dann das Lösungsmittel im Vakuum ab und destilliert den Rückstand. Man erhält 22 Teile (82,5% der Theorie) 1-(2′,4′-Dichlorphenoxy)-äthylisocyanat vom Kp 115°C/0,1 mbar, $n_D^{23} = 1{,}5303$.

Beispiele 3 bis 5

Nach den angegebenen Verfahren werden folgende Isocyanate der allgemeinen Formel I dargestellt und in Tabelle 1 niedergelegt:

Tabelle 1

| Beispiel | $R^1$ | X | $R^2$ | $R^2$ | Teile Ausgangsstoff II | Teile Ausgangsstoff IV | Endstoff Kp(°C) ($n_D^{23}$) | Ausbeute in % der Theorie |
|---|---|---|---|---|---|---|---|---|
| 3 | 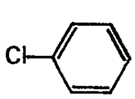 | Cl | H | H | 113 | 69 | 1,5407 | 85 |

Tabelle 1 (Fortsetzung)

| Beispiel | $R^1$ | X | $R^2$ | $R^2$ | Teile Ausgangsstoff II | Teile Ausgangsstoff IV | Endstoff Kp(°C) $(n_D^{23})$ | Ausbeute in % der Theorie |
|---|---|---|---|---|---|---|---|---|
| 4 | 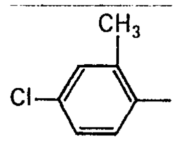 | Cl | H | H | 94 | 54 | 1,5653 | 76 |
| 5 | 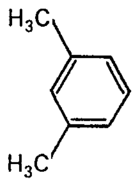 | Cl | $CH_3$ | H | 94 | 54 | 1,5040 | 71 |

Die Verwendung der erfindungsgemässen Stoffe zur Herstellung von herbiziden Wirkstoffen kann durch folgendes Beispiel verdeutlicht werden.

Beispiel 6 (Verwendung)

Zu einer Lösung von 21,8 Teilen 2′,4′-Dichlorphenoxymethylisocyanat in 150 Teilen trockenem Toluol gibt man bei 10°C 8,2 Teile N-Butin-3-yl-N-methylamin und 0,2 Teile Natriumhydrid. Nach Beendigung der exothermen Reaktion rührt man das Gemisch noch eine Stunde unter Eiskühlung nach, saugt den Niederschlag ab und wäscht gründlich mit Petroläther (40 bis 60°C) nach. Man erhält 22 Teile (73% der Theorie) N-(2′,4′-Dichlorphenoxymethyl)-N′-butin-3-yl-N′methylharnstoff vom Schmelzpunkt 116 bis 117°C.

Die herbizide Wirkung dieses Stoffes geht aus den folgenden Beispielen hervor. Zur Darstellung der herbiziden Wirksamkeit der Verbindungen führte man folgende Gewächshausversuche durch:

Als Kulturgefässe dienten Plastikblumentöpfe mit 300 cm³ Inhalt und lehmigem Sand mit etwa 1,5% Humus als Substrat. Die Samen der Testpflanzen wurden nach Arten getrennt flach eingesät und zog sie je nach Wuchsform erst bis zu einer Wuchshöhe von 3 bis 10 cm an. Einige Arten liess man in den Töpfen, in welche man die Samen einsäte. Andere wurden zunächst als Keimpflanzen getrennt angezogen und einige Tage vor der Behandlung in die Versuchsgefässe verpflanzt. Die Aufstellung der Versuche erfolgte im Gewächshaus, wobei für wärmeliebende Arten wärmere Bereiche (20 bis 35°C) und für solche gemässigter Klimate 10 bis 25°C bevorzugt wurden. Die Versuchsperiode erstreckte sich über 2 bis 4 Wochen. Während dieser Zeit wurden die Pflanzen gepflegt und ihre Reaktion auf die einzelnen Behandlungen ausgewertet. Bewertet wurde nach einer Skala von 0 bis 100. Dabei bedeutet 0 keine Schädigung oder normaler Ablauf und 100 kein Aufgang der Pflanzen bzw. völlige Zerstörung zumindest der oberirdischen Teile.

Die Ergebnisse (Tabelle 2 und 3) zeigen, dass gerade die beispielsweise aufgeführte Verbindung eine ganz beachtliche herbizide Wirkung besitzt.

Tabelle 2 – Liste der Pflanzennamen

| Botanischer Name | Deutscher Name | Englischer Name |
|---|---|---|
| Amaranthus spp. | zurückgekrümmter Fuchsschwanz | redroot pigweed |
| Chenopodium album | weisser Gänsefuss | lambsquarters |
| Euphorbia geniculate | Südamerik. Wolfmilchart | Southamerican member of the spurge family |
| Ipomea spp. | Prunkwindearten | morningglory |
| Sesbania exaltata | Turibaum | hemp sesbania (coffeeweed) |
| Xanthium pensylvanicum | Spitzklette | common cocklebur |

Tabelle 3 - Selektive herbizide Wirksamkeit von

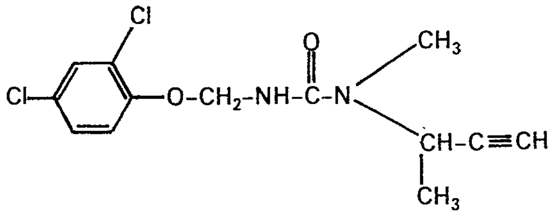

bei Nachauflaufanwendung im Gewächshaus

| Testpflanzen | Aufwandmenge kg/ha a.S. und Schädigung % 1,0 |
|---|---|
| Amaranthus spp. | 100 |
| Chenopodium album | 90 |
| Euphorbia geniculata | 100 |
| Ipomoea spp. | 100 |
| Sesbania exaltata | 100 |
| Xanthium pensylvanicum | 100 |

0 = keine Schädigung, 100 = Pflanzen abgestorben

**Patentansprüche**

1. Aryloxyalkylisocyanate der Formel

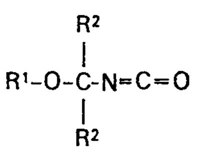

(I),

worin $R^1$ einen aromatischen Rest bezeichnet, die einzelnen Reste $R^2$ gleich oder verschieden sein können und jeweils für ein Wasserstoffatom oder einen Alkylrest mit 1 bis 18 Kohlenstoffatomen, einen cycloaliphatischen Rest, einen Aralkylrest mit 7 bis 12 Kohlenstoffatomen, oder aromatischen Rest stehen, wobei, wenn $R^1$ einen Phenylrest und ein Rest $R^2$ ein Wasserstoffatom oder eine Phenylgruppe bedeuten oder, wenn $R^1$ einen p-Tolylrest, p-Chlorphenylrest, p-Nitrophenylrest, 2,4,6-Trijodphenylrest, 2-Naphthylrest und ein Rest $R^2$ einen Phenylrest bezeichnen, die beiden Reste $R^2$ dann verschieden sind.

2. Verfahren zur Herstellung von Aryloxyalkylisocyanaten der Formel

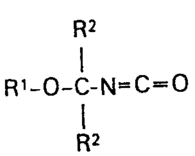

(I),

worin $R^1$ einen aromatischen Rest bezeichnet, die einzelnen Reste $R^2$ gleich oder verschieden sein können und jeweils für ein Wasserstoffatom oder einen Alkylrest mit 1 bis 18 Kohlenstoffatomen, einen cycloaliphatischen Rest, einen Aralkylrest mit 7 bis 12 Kohlenstoffatomen oder aromatischen Rest stehen, wobei, wenn $R^1$ einen Phenylrest und ein Rest $R^2$ ein Wasserstoffatom oder eine Phenylgruppe bedeuten oder, wenn $R^1$ einen p-Tolylrest, p-Chlorphenylrest, p-Nitrophenylrest, 2,4,6-Trijodphenylrest, 2-Naphthylrest und ein Rest $R^2$ einen Phenylrest bezeichnen, die beiden Reste $R^2$ dann verschieden sind, dadurch gekennzeichnet, dass man 2-Aryloxyessigsäureverbindungen der Formel

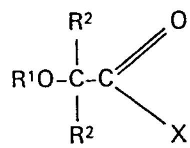

(II),

worin $R^1$ und $R^2$ die vorgenannten Bedeutungen besitzen und X ein Halogenatom, den Rest

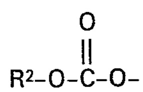

oder den Rest

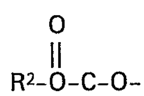

bezeichnet, mit einem Trialkylsilylazid bei einer Temperatur von −25 bis +180°C, umsetzt und aus dem Reaktionsgemisch den Endstoff I in üblicher Weise, z.B. durch fraktionierte Destillation, abtrennt.

**Claims**

1. An aryloxyalkyl isocyanate of the formula

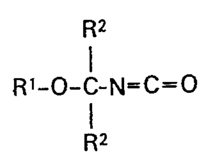

(I),

where $R^1$ is an aromatical radical, the individual radicals $R^2$ may be identical or different and each denotes hydrogen, alkyl of 1 to 18 carbon atoms, a cycloaliphatic radical, aralkyl of 7 to 12 carbon atoms, or an aromatic radical, the two radicals $R^2$ being different if $R^1$ is phenyl and one of the $R^2$'s is hydrogen or phenyl, or if $R^1$ is p-tolyl, p-chlorophenyl, p-nitrophenyl, 2,4,6-triiodophenyl or 2-naphthyl, and one of the $R^2$'s is phenyl.

2. A process for the preparation of an aryloxyalkyl isocyanate of the formula

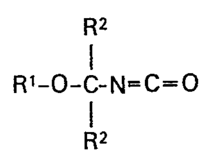

(I),

where $R^1$ is an aromatic radical, the individual radicals $R^2$ may be identical or different and each denotes hydrogen, alkyl of 1 to 18 carbon atoms, a cycloaliphatic radical, aralkyl of 7 to 12 carbon atoms, or an aromatic radical, the two radicals $R^2$ being different if $R^1$ is phenyl and one of the $R^2$'s hydrogen or phenyl, or if $R^1$ is p-tolyl, p-chlorophenyl, p-nitrophenyl, 2,4,6-triiodophenyl or 2-naphthyl, and one of the $R^2$'s is phenyl, wherein a 2-aryloxyacetic acid compound of the formula

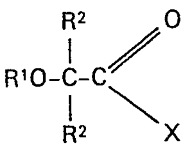

(II),

where $R^1$ and $R^2$ have the above meanings, and X is halogen,

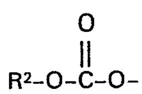

or

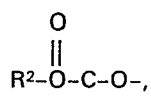

is reacted with a trialkyl silyl azide at a temperature of from −25° to +180°C, and the end product I is separated from the reaction mixture in a conventional manner, e.g. by fractional distillation.

**Revendications**

1. Isocyanates d'aryloxy-alkyle de la formule

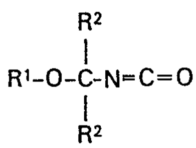

(I),

dans laquelle $R^1$ représente un reste aromatique et les restes $R^2$, qui peuvent être identiques ou différents, désignent chacun un atome d'hydrogène ou un radical alkyle en $C_1$ à $C_{18}$, un groupe cycloaliphatique, un groupe aralkyle en $C_7$ à $C_{12}$ ou un reste aromatique, les deux restes $R^2$ devant cependant être différents lorsque $R^1$ représente un groupe phényle et l'un des restes $R^2$ un atome d'hydrogène ou un groupe phényle, ou encore lorsque $R^1$ représente un groupe p-tolyle, p-chloro-phényle, p-nitro-phényle, 2,4,6-triiodo-phényle ou 2-naphthyle et l'un des restes $R^2$ un gropue phényle.

2. Procédé de préparation d'isocyanates d'aryloxy-alkyle de la formule

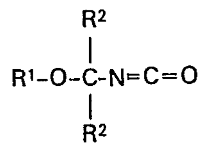

(I),

dans laquelle $R^1$ représente un reste aromatique et les restes $R^2$, qui peuvent être identiques ou différents, désignent chacun un atome d'hydrogène ou un radical alkyle en $C_1$ à $C_{18}$, un groupe cycloaliphatique, un groupe aralkyle en $C_7$ à $C_{12}$ ou un reste aromatique, les deux restes $R^2$ devant cependant être différents lorsque $R^1$ représente un groupe phényle et l'un des restes $R^2$ un atome d'hydrogène ou un groupe phényle, ou encore lorsque $R^1$ représente un groupe p-tolyle, p-chloro-phényle, p-nitro-phényle, 2,4,6-triiodo-phényle ou 2-naphthyle et l'un des restes $R^2$ un groupe phényle, caractérisé en ce que l'on fait réagir un dérivé de l'acide 2-aryloxy-acétique de la formule

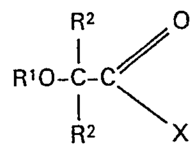

(II),

dans laquelle $R^1$ et $R^2$ possèdent les significations définies et X désigne un atome d'halogène, un groupe

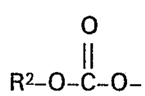

ou un groupe

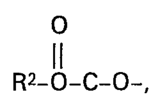

à une température comprise entre −25 et +180°C, avec un trialkylsilyl-azide, le produit final I étant isolé du mélange réactionnel par une voie usuelle telle qu'une distillation fractionnée.